(19) Europäisches Patentamt
European Patent Office
Office européen des brevets

(11) Veröffentlichungsnummer: **0 000 023**
**B1**

# (12) EUROPÄISCHE PATENTSCHRIFT

(21) Anmeldenummer: **78100035.1**

(22) Anmeldetag: **01.06.78**

(51) Int. Cl.³: **C 07 C1 31/00,**
A 01 N 47/34

## (54) Omega-substituierte Pentyl-harnstoff-Derivate, Verfahren zu ihrer Herstellung sowie ihre Verwendung als Fungizide

(30) Priorität: **04.06.77 DE 2725315**
**24.12.77 DE 2758108**

(43) Veröffentlichungstag der Anmeldung:
**20.12.78 Patentblatt 78/01**

(45) Bekanntmachung des Hinweises auf die Patenterteilung:
**23.07.80 Patentblatt 80/15**

(84) Benannte Vertragsstaaten:
**BE CH DE FR GB NL SE**

(56) Entgegenhaltungen:
**DE - A - 2 312 956**

(73) Patentinhaber: **Bayer AG**
**Zentralbereich Patente, Marken und Lizenzen**
**D - 5090 Leverkusen 1, Bayerwerk (DE)**

(72) Erfinder: **Brandes, Wilhelm, Dr.**
**Eichendorffstrasse 3**
**D - 5653 Leichlingen (DE)**
**Daum, Werner, Dr.**
**Buschstrasse 167**
**D - 4150 Krefeld 1 (DE)**

**Bayer AG**

**D - 5653 Leichlingen (DE)**

EP 0 000 023 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Omega-substituierte Pentyl-harnstoff-Derivate, Verfahren zu ihrer Herstellung sowie ihre Verwendung als Fungizide

Die vorliegende Erfindung betrifft neue $\omega$-substituierte Pentyl-harnstoff-Derivate, mehrere Verfahren zu ihrer Herstellung sowie ihre Verwendung als Fungizide.

Wie bereits lange bekannt ist, werden als Fungizide in der Landwirtschaft und im Gartenbau insbesondere das Zink-äthylen-1,2-bis-dithiocarbamidat und das N-Trichlormethylthio-tetrahydrophthalimid verwendet; die genannten Verbindungen besitzen unter den Handelsprodukten eine große Bedeutung (vgl. R. Wegler, "Chemie der Pflanzenschutz- und Schädlingsbekämpfungsmittel", Band 2, Seiten 65 und 108, Berlin/Heidelberg/New York (1970)). Die Wirkung bei niedrigen Aufwandkonzentrationen ist jedoch nicht immer befriedigend. Auch sind diese Fungizide nicht curativ einsetzbar.

Weiterhin bekannt ist die fungizide Wirkung von einigen Isonitrosocyanacetamid-Derivaten (vgl. hierzu die DT—OSS 1 693 052, 2 118 317, 2 312 956, 2 350 910, 2 436 654, 2 436 655, 2 603 643 und 2 635 697 und die US—PSS 3 625 987, 3 769 423, 3 919 284, 3 954 992 und 3 957 847. Auch hier ist die Wirksamkeit bei niedrigen Aufwandmengen nicht zuverlässig. und bei normalen Konzentrationen werden Pflanzenschäden gesehen.

Es wurden nun als neue Stoffe die $\omega$-substituierten Pentyl-harnstoff-Derivate der allgemeinen Formel

$$NC-\underset{\underset{\displaystyle N-O-R}{\|}}{C}-CO-NH-CO-NH-(CH_2)_5-Q \qquad (I)$$

in welcher

R für $R^1$, $CO-R^2$, $CO-NH-R^3$ oder für $CO-OR^4$ steht, wobei

$R^1$ für unsubstituiertes Alkyl mit 1 bis 10 Kohlenstoffatomen steht; ferner für substituiertes Alkyl mit 1 bis 4 Kohlenstoffatomen steht, welches als Substituenten eine Vinylgruppe, eine Alkinylgruppe mit bis zu 4 Kohlenstoffatomen, eine Alkylcarbonylgruppe mit 2 bis 5 Kohlenstoffatomen, eine Alkoxycarbonylgruppe mit 2 bis 5 Kohlenstoffatomen, eine Alkenoxycarbonyl- oder Alkinoxycarbonyl-Gruppe mit 4 bis 5 Kohlenstoffatomen, eine Aminocarbonyl-, N-Alkylaminocarbonyloder N-Cycloalkylaminocarbonyl-Gruppe mit jeweils bis zu 7 Kohlenstoffatomen, oder eine N-Phenylaminocarbonylgruppe, die am Phenylrest gegebenenfalls niedere Alkylgruppen und/oder Chloratome als weitere Substituenten besitzen kann, enthält; ferner für Benzyl steht, welches im aromatischen Teil durch Methyl-, Methoxy-, Methylendioxy-, Nitro-, Trifluormethyl-, Benzoyl-, Mono- oder Dichlorbenzoyl-, Phenyl- oder Phenoxy-Gruppen oder durch 1 bis 4 Chloratome substituiert sein kann; und

$R^2$ für einen geraden oder verzweigten, gesättigten oder ungesättigten Kohlenwasserstoffrest mit bis zu 8 Kohlenstoffatomen steht, welcher durch Chlor- oder Bromatome bzw. durch eine Cyangruppe substituiert sein kann, und

$R^3$ für einen geraden oder verzweigten Alkylrest mit bis zu 11 Kohlenstoffatomen steht, welcher durch eine Cyangruppe oder durch einen Carbonyloxyalkylrest mit bis zu 5 Kohlenstoffatomen substituiert sein kann, oder für einen Phenylrest steht, der durch Methyl-, Nitro- oder Trifluoromethyl-Gruppen bzw. durch Chloratome substituiert sein kann, und

$R^4$ für eine gesättigte oder ungesättigte aliphatische Gruppe mit bis zu 4 Kohlenstoffatomen steht, und

Q für CN, $CO-NH_2$, COOH oder $CO-OR^5$ steht, wobei

$R^5$ eine Alkyl-Gruppe mit 1 bis 4 Kohlenstoffatomen bedeutet,

gefunden. Sie besitzen starke fungizide Eigenschaften.

Die erfindungsgemäßen Stoffe können als Oximderivate in zwei verschiedenen geometrischen Strukturen vorliegen:

$$NC-\underset{\underset{\displaystyle N-O-R}{\|}}{C}-CO-NH-CO-NH-(CH_2)_5-Q \qquad \text{bzw.} \qquad NC-\underset{\underset{\displaystyle R-O-N}{\|}}{C}-CO-NH-CO-NH-(CH_2)_5-Q$$

(Ia) (Ib)

Im folgenden wird auf die Angabe der Räumlichen Struktur verzichtet; für die Zwecke der vorliegenden Anmeldung sollen die angegebenen Formeln (Ia) in jedem Fall auch die entsprechende Formel gemäß der räumlichen Struktur (Ib) mitumfassen.

Weiterhin wurde gefunden, daß man $\omega$-substituierte Pentyl-harnstoff-Derivate der Formel Ia bzw. Ib erhält, wenn man

a) ein 2-Cyan-2-alkoxyimino-acetamid der Formel

$$NC-\underset{\underset{N-O-R^1}{\|}}{C}-CO-NH_2 \qquad (II)$$

in welcher

$R^1$ die oben angegebene Bedeutung besitzt, in Gegenwart einer starken Base mit einem $\omega$-substituierten Pentylisocyanat der Formel

$$O{=}C{=}N-(CH_2)_5-Q \qquad (III)$$

in welcher

Q die oben angegebene Bedeutung besitzt, umsetzt, oder

b) den 1-(5-substituierten Pentyl)-3-(2-cyan-2-oximino-acetyl)-harnstoff der Formel

$$NC-\underset{\underset{N-OH}{\|}}{C}-CO-NH-CO-NH-(CH_2)_5-Q \qquad (IV)$$

in welcher

Q die oben angegebene Bedeutung besitzt,

in Gegenwart eines Säurebindemittels, bzw. ein Alkali-, Erdalkali- oder Ammoniumsalz des Harnstoffs der Formel IV mit einem Alkylierungsmittel der Formel

$$X-R^1 \qquad (V)$$

in welcher

$R^1$ die oben angegebene Bedeutung besitzt, und

X für eine "Abgangsgruppe" wie Chlor, Brom, Jod, Alkoxy-, Alkyl- bzw. Arylsulfonyl steht, und ferner für die Gruppe $(CH_3O-)_2PO-O$ steht, wenn $R^1$ für Methyl steht,

umsetzt, bzw.

c) den Harnstoff der Formel IV mit einem Säurederivat der Formel

$$T-CO-R^2 \qquad (VI)$$

in welcher

$R^2$ die oben angegebene Bedeutung besitzt und

T für Chlor, Brom, Jod oder die Gruppe $R^2-CO-O$ steht, umsetzt, bzw.

d) den Harnstoff der Formel IV mit einem Isocyanat der Formel

$$OCN-R^3 \qquad (VII)$$

oder mit einem Carbamidsäurehalogenid der Formel

$$Y-CO-NH-R^3 \qquad (VIII)$$

in welchen Formeln

$R^3$ die oben genannte Bedeutung besitzt und

Y für Chlor, Brom oder Jod steht,

umsetzt, bzw.

e) den Harnstoff der Formel IV mit einem Kohlensäureester der Formel

$$Z-CO-O-R^4 \qquad (IX)$$

in welcher

$R^4$ die oben angegebene Bedeutung besitzt und

Z für Chlor oder den Rest $R^4-O-CO-O$ steht,

umsetzt;

weiterhin kann man noch diejenigen Verbindungen der Formel I, in denen R für $R^1$ und Q für die Gruppe $CO-OR^5$ steht, dadurch erhalten, daß man gemäß einer weiteren Verfahrensvariante

f) eine nach Verfahren b) synthetisierte Carbonsäure der Formel

$$NC-\underset{\underset{N-O-R^1}{\|}}{C}-CO-NH-CO-NH-(CH_2)_5-CO-OH \qquad (X)$$

in welcher
$R^1$ die weiter oben angegebene Bedeutung besitzt,
in Gegenwart eines Säurebindemittels, bzw. eines Alkali-, Erdalkali- oder Ammonium-Ions, mit einem Alkylierungsmittel der Formel

$$X—R^5 \qquad (XI)$$

in welcher
X und $R^5$ die oben genannte Bedeutung besitzen, umsetzt.

Die erfindungsgemäßen Verbindungen zeigen eine gute fungizide Wirkung. Sie sind protektiv, curativ und sogar eradikativ anwendbar, außerdem haben sie systemische und/oder locosystemische Eigenschaften. Überraschenderweise zeigen sie eine bessere Pflanzenverträglichkeit als die nach dem Stand der Technik bekannten Isonitrosocyanacetamid-Derivate. Gegenüber den Dithiocarbamidäten und dem N-Trichlormethylthio-tetrahydrophthalimid besitzen sie den Vorteil curativer und eradikativer Wirkung.

Die erfindungsgemäßen Verbindungen stellen schon wegen der vielen Möglichkeiten ihrer überlegenen biologischen Anwendung eine wertvolle Bereicherung der Technik dar. Ein weiterer wesentlicher Gesichtspunkt dieser Erfindung ist, daß neue Wirkstoffe mit für die Praxis wertvollen Eigenschaften zu einer Zeit zur Verfügung gestellt werden, da durch Resistenzerscheinungen älterer Wirkstoffe ein ausgesprochener Bedarf nach neuen Fungiziden besteht.

Verwendet man 1-(5-Cyanpentyl)-3-(2-cyan-2-oximino-acetyl)-harnstoff und Benzylchlorid als Ausgangsstoffe, sowie Äthyldiisopropylamin als Protonenakzeptor, so kann der Reaktionsverlauf durch das folgende Formelschema wiedergegeben werden:

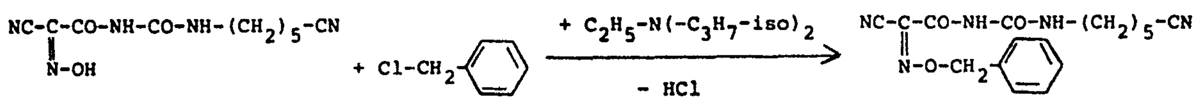


Die erfindungsgemäßen Verbindungen sind durch die Formel I definiert. In ihr steht R vorzugsweise für $R^1$, und $R^1$ vorzugsweise für Alkyl mit 1 bis 6 C-Atomen; ferner für solches Alkyl mit 1 bis 2 C-Atomen, welches durch eine Vinylgruppe, durch eine Alkylcarbonylgruppe mit 2 bis 5 C-Atomen, durch Alkoxycarbonyl mit 2 bis 5 C-Atomen, durch Aminocarbonyl, durch N-Alkylaminocarbonyl oder N-Cycloalkylaminocarbonyl mit bis zu 7 C-Atomen, durch N-Phenylaminocarbonyl, das im Phenylrest niedere Alkylsubstituenten und/oder Chloratome enthalten kann, substituiert ist, oder für Benzyl, welches im aromatischen Teil Methyl-, Methoxy-, Methylendioxy-, Nitro- bzw. Trifluoromethylgruppen bzw. 1 bis 2 Chloratome als Substituenten tragen kann.

Besonders bevorzugt wird R für folgende Strukturelemente von $R^1$: Methyl, Äthyl, Methoxycarbonylmethyl, 1-(Methoxycarbonyl)-äthyl-1, tert.-Butylcarbonylmethyl- und Aminocarbonylmethyl.

Ein interessanter Wirkstoff ist diejenige Verbindung aus der allgemeinen Formel I, in welcher R für Methyl steht.

Zur Herstellung der Ausgangsprodukte ist zu vermerken:

Die nach Verfahren a) benötigten 2-Cyan-2-alkoxyimino-acetamide der Formel II sind größtenteils bekannte Verbindungen (vgl. hierzu Ber. *54*, 1342 (1911), ferner DT—OS 2 312 956 und die Deutsche Patentanmeldung P 2 623 847 vom 28.5.1976 [Le A 17 136]; dasselbe gilt für die umzusetzenden Isocyanate (III) (Liebigs Ann. Chem. *562*, 104 (1949) und DT—OS 1 913 273).

Zu nennen sind hier die folgenden Ausgangsstoffe:
2-Cyan-2-methoxyimino-acetamid, 2-Cyan-2-äthoxyimino-acetamid, 2-Cyan-2-octyloxyimino-acetamid;
ω-Cyanpentylisocyanat, ω-Methoxycarbonyl-pentylisocyanat, ω-Isopropoxycarbonyl-pentylisocyanat und ω-Butoxycarbonylpentylisocyanat.

Die als Ausgangstoffe gemäß den Verfahrensvarianten b) bis c) benötigten 1-(5-substituierte Pentyl)-3-(2-cyan-2-oximinoacetyl)-harnstoffe der Formel IV sind bislang noch nicht bekannt. Sie werden hergestellt, indem man z.B. ω-Cyanpentylisocyanat (vgl. Liebigs Ann.Chem. *562*, 104 (1949), Cyanpentylcarbamidsäurechlorid (DT—OS 2 626 828 [Le A 17 135]), ω-Isocyanato-capronsäurealkylester (vgl. DT—OS 1 913 273), ω-Phenoxycarbonylamino-capronsäure (vgl. DT—OS 1 720 606, ferner Chem. Abstr. *71*, 50689 (1969)) oder ω-Isocyanatocapronsäurechlorid (DT—AS 1 222 919) gelöst oder suspendiert in einem inerten Lösungsmittel, wie z.B. Toluol, mit Ammoniak umsetzt. Wird ω-Phenoxycarbonylamino-capronsäure als Ausgangsstoff eingesetzt, erhält man 5-Carboxypentylharnstoff als Ammoniumsalz. Durch Ansäuern mit einer Mineralsäure entsteht die freie Ureidopentylcarbonsäure. Der ω-substituierte 1-Pentyl-harnstoff wird nun mit überschüssiger Cyanessigsäure in Gegenwart von Dicarbonsäureanhydrid zum 1-(5-substituierten Pentyl)-3-(2-cyanacetyl)-harnstoff umgesetzt; als Lösungsmittel kann Toluol dienen. In einer dritten Stufe wird der genannte Harnstoff in Wasser oder Gemischen von Wasser mit Methanol. Äthanol, Propanol, Glycolmonomethyläther, Glycolmonoäthyläther, Acetonitril, Dioxan oder Tetrahydrofuran mit einem Salz der salpetrigen Säure bei einer Temperatur von 20 bis 70°C, vorzugsweise bei 40 bis 50°C, durch Zugabe einer organischen

Carbonsäure, wie z.B. Essigsäure oder einer Mineralsäure, wie z.B. Schwefelsäure oder auch durch Mischungen von organischen Carbonsäuren mit Mineralsäuren auf einen pH-Wert von 4 bis 6, vorzugsweise auf einen pH-Wert von 5 bis 5,6 gebracht. Nach etwa 2 Stunden ist die Oximierung beendet. Die Reaktionsmischung wird durch Hinzufügen weiterer Mineralsäure bei einer Temperatur, die zwischen dem Gefrierpunkt der Lösung und etwa 30°C liegt, vorzugsweise zwischen +2 und 10°C liegt, auf einen pH-Wert von 1,8 bis 3,5, vorzugsweise von etwa 2, eingestellt. Der 1-(5-subsituierte Pentyl)-3-(2-cyan-2-oximino-acetyl)-harnstoff der Formel IV wird entweder durch Verdünnen der Reaktionsmischung mit Wasser abgeschieden und nach dem Abtrennen des Produkts mit Wasser gewaschen und getrocknet, oder man nimmt die Verbindung in einem Lösungsmittel, wie Äthylacetat, Methylacetat, Äthylformiat oder Methylpropionat auf, wäscht die erhaltene Lösung des Reaktionsproduktes mit Wasser aus, trocknet mit z.B. Natriumsulfat und fällt durch Zugabe von Petroläther das Produkt IV aus, oder man dampft die Lösung ein.

Schließlich kann der Harnstoff der Formel IV auch durch Einwirkung eines Esters der salpetrigen Säure, wie z.B. Isoamylnitrit, auf einen 1-(5-substituierten Pentyl)-3-(2-cyanacetyl)-harnstoff erhalten werden.

Die für das Verfahren b) benötigten Alkylierungsmittel der Formel V sind größtenteils bekannte, laboratoriumsübliche Verbindungen. Soweit sie noch nicht beschrieben sind, können sie nach prinzipiell bekannten Verfahren erhalten werden. Als Beispiele für Ausgangsstoffe der Formel V sind zu nennen: Dimethylsulfat, Diäthylsulfat, 2-Brombutan, 1-Jodpentan, 1-Bromhexan, 1-Bromdecan, Allylchlorid, Methallylchlorid, 1-Chlorbuten-2, Propargylchlorid, 1-Chloraceton, 1-Brom-3,3-dimethyl-butanon-2, 2-Brom-4,4-dimethyl-pentanon-3, Bromessigsäuremethylester, Chloressigsäure-sek.-butylester, 2-Bromvaleriansäurebutylester, 4-Chlor-buttersäure-äthylester, Chloracetamid, N-Methyl-chloracetamid, N-Äthylchloracetamid, N-sek.-Butyl-chloracetamid, N-Hexylchloracetamid, N-Cyclopropyl-chloracetamid, N-Cyclopentylchloracetamid, N-Cyclohexylchloracetamid, Bromacetanilid, N-2-Äthylphenyl-, N-2-Methylphenyl-, N-2-Isopropylphenyl-, N-2-tert.-Butylphenyl-, N-3-Methylphenyl-, N-4-Methylphenyl-, N-2,6-Dimethylphenyl-, N-2-Chlorphenyl-, N-3-Chlorphenyl-, N-4-Chlorphenyl-, N-3,4-Dichlorphenyl-, N-3,5-Dichlorphenyl- und N-2-Methyl-4-chlorphenyl-chloracetamid, -2-chlorpropionamid und -2-chlorbutyramid, Benzylchlorid, 2-, 3- oder 4-Xylylchlorid, 2-, 3- oder 4-Methoxybenzylbromid, 3,4-Methylendioxybenzylchlorid, 3-Nitrobenzylchlorid, 4-Nitrobenzylchlorid, 4-Trifluormethylbenzylchlorid, 2-, 3- oder 4-Chlorbenzylchlorid, 3,4-Dichlorbenzylchlorid, 2-Phenylbenzylchlorid, 4-Phenyl-benzylchlorid, 4-Benzoylbenzylchlorid, 4-(2-, 3- oder 4-Chlorbenzoyl)-benzylchlorid, 4-(2,3-, 2,4-, 2,5-, 2,6-, 3,4- oder 3,5-Dichlorbenzoyl)-benzylchlorid, 2-, 3- oder 4-Phenoxybenzylchlorid, ar.-Tetrachlor-o-, Tetrachlor-m- und Tetrachlor-p-xylylchlorid.

Die für die Umsetzung nach Verfahren c) benötigten Säureanhydride und Säurehalogenide der Formel VI sind laboratoriumsübliche Verbindungen. Zu nennen sind hier:

Acetanhydrid, Propionsäureanhydrid, Chloracetylchlorid, Dichloracetylchlorid, Trichloracetylchlorid 2,3-Dibrompropionsäurechlorid, Isobuttersäurechlorid, 2-Chlorisobuttersäureclorid, Valeriansäurechlorid, Isovaleriansäurechlorid, 2-Äthylhexansäurechlorid, Cyanessigsäurechlorid, Acrylsäurechlorid, Acrylsäurechlorid, Methacrylsäurechlorid, Crotonsäurechlorid.

Die für die Umsetzung nach Verfahren d) benötigten Isocyanate der Formel VII bzw. die Carbamidsäurehalogenide der Formel VIII sind ebenfalls bekannte Verbindungen; die Herstellung der Carbamidsäurehalogenide erfolgt durch Halogenwasserstoff-Addition an die Isocyanate der Formel VI.

Zu nennen sind hier die folgenden Verbindungen:

Methyl-, Äthyl-, Propyl-, Butyl-, Isobutyl-, Hexyl-, Decyl-isocyanat oder -carbamidsäurechlorid,
$\omega$-Cyanäthyl-isocyanat,
1-Cyan-1-methyl-äthyl-isocyanat,
$\omega$-Cyanpropyl-isocyanat,
$\omega$-Cyanpentyl-isocyanat,
$\omega$-Cyanhexyl-isocyanat,
$\omega$-Cyanoctyl-isocyanat,
$\omega$-Cyannonyl-isocyanat,
$\omega$-Cyandecyl-isocyanat,
$\omega$-Cyanundecyl-isocyanat,
Methoxycarbonylmethyl-isocyanat,
Äthoxycarbonylmethyl-isocyanat,
Butoxycarbonyläthyl-isocyanat,
Isobutoxycarbonyläthyl-isocyanat,
1-Methoxycarbonyl-1-methyl-äthyl-isocyanat,
1-Propoxycarbonyl-1-methyl-äthyl-isocyanat,
1-Äthoxycarbonyl-1-äthyl-äthyl-isocyanat,
1-Isobutoxycarbonyl-1-äthyl-äthyl-isocyanat,
Methoxycarbonyl-propyl-isocyanat,
Methoxycarbonyl-pentyl-isocyanat,
Isopropoxycarbonyl-pentyl-isocyanat,
sek.-Butyloxycarbonyl-pentyl-isocyanat,

2-Äthoxycarbonyl-2-äthyl-butyl-isocyanat,
γ-Äthoxycarbonyl-octyl-isocyanat,
Methoxycarbonyl-decyl-isocyanat,
Äthoxycarbonyl-decyl-isocyanat,
Propoxycarbonyl-decyl-isocyanat,
Butoxycarbonyl-decyl-isocyanat,
Methoxycarbonyl-undecyl-isocyanat,
Phenylisocyanat, 2- und 4-Tolylisocyanat, 3- und 4-Nitrophenylisocyanat, 2-, 3- und 4-Chlorphenylisocyanat, 3,4-Dichlorphenylisocyanat, 3,5-Dichlorphenylisocyanat und 2-Trifluormethylisocyanat.

Die nach dem Verfahren e) benötigten Kohlensäureester sind ebenfalls bekannte Verbindungen. Zu nennen sind:
Dimethylpyrocarbonat, Diäthylpyrocarbonat, Chlorameisensäureäthylester, Chlorameisensäuremethylester, Chlorameisensäureisopropylester, Chlorameisensäure-sek.-butylester, Chlorameisensäureisobutylester, Chlorameisensäureallylester und Chlorameisensäuremethallylester.

Zur Herstellung der erfindungsgemäßen Verbindungen gemäß Verfahren a) wird das 2-Cyan-2-alkoximinoacetamid in einem indifferenten wasserfreien Lösungsmittel, wie Dioxan, Tetrahydrofuran oder Diisopropyläther, mit z.B. Natriumhydrid oder Kalium-tert.-butylat in das Anion des Amids übergeführt und anschließend mit ω-substituiertem Pentylisocyanat bei mäßig erhöhter Temperatur umgesetzt. Nach beendeter Reaktion wird in der Kälte mit einer organischen Carbonsäure schwach angesäuert, danach durch Wasserzusatz das erfindungsgemäße Reaktionsprodukt ausgefällt bzw. die Lösung des Reaktionsproduktes im organischen Lösungsmittel ausgewaschen und vorsichtig eingedampft.

Zur Herstellung derjenigen erfindungsgemäßen Verbindungen der Formel I, bei welchen R für $R^1$ steht, gemäß Verfahren b) arbeitet man vorzugsweise in Gegenwart von polaren Lösungsmitteln wie Dimethylsulfoxid, Dimethylformamid, Dimethylacetamid, Aceton, Methyläthylketon, Methylenchlorid, Chloroform, Chlorbenzol, Toluol, Dioxan, Tetrahydrofuran, Acetonitril, Benzonitril oder Essigsäureäthylester.

Als Säurebindemittel können bei Verfahren b) alle üblichen Halogenwasserstoff-Akzeptoren verwendet werden. Hierzu gehören Alkalihydroxide, Alkalicarbonate und andere geeignete Alkalisalze. Zu nennen sind z.B. Natriumcarbonat, Natriumbicarbonat, Borax (Dinatriumtetraborat) und Trilithiumphosphat. Arbeitet man in Gegenwart von Wasser so kann durch Zugabe von Natronlauge neutralisiert werden. Ferner können organische Säurebinder Verwendung finden, wie z.B. tertiäre Amine. Zu nennen sind hier Triäthylamin, Dimethylbenzylamin, Dimethylanilin, Pyridin, Picolin, Chinolin, Äthyldiisopropylamin und Äthyldicyclohexylamin.

Die Reaktionstemperaturen können bei Verfahren b) in einem größeren Bereich variiert werden. Im allgemeinen arbeitet man zwischen etwa —50 und etwa +120°C, vorzugsweise zwischen —5 und +80°C.

Die Reaktionen können auch in Mischungen aus Wasser und einem wassermischbaren organischen Lösungsmittel durchgeführt werden oder in heterogenen Systemen, bestehend aus Wasser und einem mit Wasser nicht mischbaren oder nur teilweise mischbaren Lösungsmittel; hierbei liegt der Temperaturbereich der Umsetzung zwischen dem Gefrierpunkt des Wassers bzw. dem Erstarrungspunkt der wäßrigen Lösung und ca. 100°C, vorzugsweise bei —5° bis +80°C.

Verwendet man jedoch bei Verfahren b) die Salze des Harnstoffs der Formel IV als Ausgangsprodukte, so muß man die Reaktion bei niedrigen Temperaturen, vorzugsweise bei —30°C bis —10°C, durchführen und nach beendeter Reaktion schwach ansäuern.

Reaktionstemperaturen und Reaktionsdauer bei Verfahren b) werden durch die Aktivität der Ausgangsprodukte der Formel V bestimmt. Zweckmäßigerweise setzt man den Mischungen vor der Reaktion eine kleine Menge eines Jodids zu, wenn man nicht gerade eine Verbindung der Formel V mit Jod als Abgangsgruppe einsetzt. Hierdurch wird die Reaktionsgeschwindigkeit erhöht und die Gefahr der Bildung von Verbindungen mit Nitronstruktur vermindert (vgl. dazu Houben-Weyl, "Methoden der organischen Chemie", Band 10/4, Stuttgart (1968)).

Die beim Verfahren b) gegebenen Erläuterungen gelten entsprechend auch für die Verfahrensvarinate f), wenn eine Carbonsäure der Formel X zu einem solchen erfindungsgemäßen Produkt der Formel I umgesetzt werden soll, in welcher R für $R^1$ und Q für die Gruppe $CO—OR^5$ steht.

Die Herstellung der erfindungsgemäßen Verbindungen der Formel I, in welcher R für $CO—R^2$ steht, nach Verfahren c) wird in einem wasserfreien organischen Lösungsmittel in einem Temperaturbereich zwischen —50°C und +120°C, vorzugsweise bei 0 bis 50°C, vorgenommen, wobei man ein tert. Amin zur Bindung der Säure einsetzt.

Das erfindungsgemäße Verfahren d) wird zweckmäßigerweise in einem Verdünnungsmittel vorgenommen. Als solches kommen alle inerten organischen Lösungsmittel infrage. Vorzugsweise verwendet man Dimethylsulfoxid, Dimethylformamid, Dimethylacetamid und Essigsäureäthylester, ferner Ketone, wie z.B. Aceton, Mehyl-äthyl-keton und Diäthylketon, ferner Äther, wie z.B. Tetrahydrofuran, chlorierte Kohlenwasserstoffe, wie z.B. Methylenchlorid und Chloroform, Nitrile, wie z.B. Acetonitril und Benzonitril und Aromaten, wie z.B. Toluol and Chlorbenzol.

Als Hilfsstoffe können beim Verfahren d) basische Katalysatoren Verwendung finden; so z.B. tertiäre Amine, wie Triäthylamin oder Pyridin, weiterhin Zinn-2-äthyl-hexanoat. An Stelle der Isocyanate der Formel VII können jeweils die entsprechenden Carbamidsäurehalogenide der Formel VIII Verwendung finden. In diesem Fall ist zusätzliches tertiäres Amin zur Bindung der bei der Reaktion frei werdenden Halogenwasserstoffsäure erforderlich.

Die Reaktionstemperaturen können beim Verfahren d) in einem größeren Bereich variiert werden. Im allgemeinen arbeitet man im Bereich zwischen —20 und +120°C, vorzugsweise zwischen +10 und 70°C.

Die Herstellung der erfindungsgemäßen Verbindungen der Formel I, in welcher R für CO—O—$R^4$ steht, nach Verfahrensschritt e) erfolgt analog der Arbeitsweise nach Verfahren b).

Je nach Arbeitsbedingungen fallen die erfindungsgemäßen Wirkstoffe kristallin aus oder sie bleiben im organischen Lösungsmittel gelöst und können dann nach Auswaschen der Lösung mit Wasser durch vorsichtiges Einengen der Lösung oder durch Zugabe wenig polarer organischer Lösungsmittel, wie Tetrachlorkohlenstoff, Cyclohexan oder Dibutyläther abgeschieden werden.

Sind die erfindungsgemäßen Verbindungen in einem mit Wasser mischbaren Lösungsmittel gelöst, so können sie auch durch Zugabe von Wasser ausgefüllt werden. Sowie es die besonderen Bedingungen der Aufarbeitungsprozesse erlauben, sollen die Lösungen der erfindungsgemäßen Wirkstoffe bzw. die noch lösungsmittelfeuchten Suspensionen der Wirkstoffe schwachsauer eingestellt werden.

Die erfindungsgemäßen Verbindungen zersetzen sich zum Teil bei höherer Temperatur; in diesen Fällen können die Schmelzpunkte nur mit geringer Genauigkeit oder überhaupt nicht ermittelt werden. Das Vorliegen bestimmter Strukturelemente ist aus den NMR-Spektren zu entnehmen. Auch zeigen die IR-Spektren charakteristische Absorptionsbanden.

Die erfindungsgemäßen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe können angewandt werden gegen parasitäre Pilze, die oberirdische Pflanzenteile befallen oder die Pflanzen vom Boden her angreifen, sowie gegen samenübertragbare Krankheitserreger. Sie können somit auch zur Boden- und zur Saatgut-Behandlung benutzt werden.

Die Wirkstoffe zeigen insbesondere eine hohe protektive und kurative Wirksamkeit gegen Phycomyceten. Daneben sind gute Wirkungen gegen Mycosphaerella- und Rhizoctonia-Arten und gegen Rostpilze festzustellen.

Die erfindungsgemäßen Wirkstoffe weisen nicht nur die guten Eigenschaften hervorragender Handelspräparate auf, sondern besitzen darüber hinaus noch erhebliche Vorteile. Diese liegen in erster Linie in der Fähigkeit der erfindungsgemäßen Stoffe, in die Pflanze einzudringen. Sie können aufgenommen werden von der Saatgutoberfläche, von den Wurzeln und auch von oberirdischen Pflanzenorganen nach äußerlichen Applikationen. Auch besitzen sie die vorteilhafte Fähigkeit, locosystemisch zur Wirkung zu kommen, d.h. eine Tiefenwirkung im Pflanzengewebe auszuüben und dabei pilzliche Krankheitserreger zu eliminieren, die bereits in das Gewebe der Wirtspflanze eingedrungen sind.

In einem bestimmten Konzentrationsbereich sind einige der erfindungsgemäßen Stoffe auch als Wachstumsregulatoren für Pflanzen wirksam.

Die erfindungsgemäßen Wirkstoffe können in die üblichen Formulierungen übergeführt werden, wie Lösungen, Emulsionen, Suspensionen, Pulver, Pasten und Granulate. Diese werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, Benzol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Dichlordifluormethan oder Trichlorfluormethan; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als Emulgiermittel; nichtionogene und anionische Emulgatoren, wie Polyoxyäthylen-Fettsäure-Ester, Polyoxyäthylen-Fettalkohol-Äther, z.B. Alkylaryl-polyglycol-Äther, Alkylsulfonate, Alkylsulfate, Arylsulfonate sowie Eiweißhydrolysate; als Dispergiermittel: z.B. Lignin, Sulfitablaugen und Methylcellulose.

Zusätzlich zu den obigen Formulierungsmöglichkeiten ist zu bemerken, daß die erfindungsgemäßen Stoffe zusammen mit Saccharose, Dextrose, Dextrinen, mit wasserfreiem Calciumsulfat oder Calciumsulfat-hemihydrat, sowie mit Carbonsäuren, wie z.B. Fumarsäure oder 4-Hydroxylbenzoesäure, oder auch mit schwach sauren Ionenaustauschern zusammen formuliert werden können.

Die erfindungsgemäßen Wirkstoffe können in den Formulierungen in Mischungen mit anderen bekannten Wirkstoffen vorliegen, wie Fungiziden, Insektiziden, Akariziden, Nematiziden, Herbiziden, Schutzstoffe gegen Vogelfraß, Wuchsstoffen, Pflanzennährstoffen und Bodenstrukturverbesserungsmitteln.

Die Formulierungen enthalten im allgemeinen zwischen 0,1 und 95 Gewichtsprozent Wirkstoff, vorzugsweise zwischen 0,5 und 90 %.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Spritzen, Sprühen, Stäuben, Streuen, Trockenbeizen, Feuchtbeizen, Naßbeizen, Schlämmbeizen oder Inkrustieren.

Bei der Verwendung als Blattfungizide kömen die Wirkstoffkonzentrationen in den Anwendungsformen in einem größeren Bereich variiert werden. Sie liegen im allgemeinen zwischen 0,5 und 0,0005 Gewichtsprozenten, vorzugsweise zwischen 0,2 und 0,001.

Bei der Saatgutbehandlung werden im allgemeinen Wirkstoffmengen von 0,01 bis 50 g je Kilogramm Saatgut, vorzugsweise 0,5 bis 5 g, benötigt.

Zur Bodenbehandlung sind Wirkstoffmengen von 1 bis 1000 g je cbm Boden, vorzugsweise von 10 bis 200 g, erforderlich.

Die Anwendungsmöglichkeiten erläutern die folgenden Verwendungsbeispiele:

### Beispiel A

Phytophthora-Test (Tomaten) / Protektiv

| | |
|---|---|
| Lösungsmittel | 4,7 Gewichtsteile Aceton |
| Emulgator | 0,3 Gewichtsteile Alkylarylpolyglykoläther |
| Wasser | 95 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20°C und einer relativen Luftfeuchtigkeit von 70 % im Gewächshaus. Anschließend werden die Tomatenpflanzen mit einer wäßrigen Sporensuspension von Phytophthora infestans inokuliert. Die Pflanzen werden in eine Feuchtkammer mit einer 100 %igen Luftfeuchtigkeit und einer Temperatur von 18 bis 20°C gebracht.

Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.

Wirkstoff, Wirkstoffkonzentration und Ergebnisse gehen aus der nachfolgenden Tabelle hervor:

TABELLE A

Phytophthora-Test (Tomaten) / Protektiv

| Wirkstoff | Befall in % bei einer Wirkstoffkonzentration von 0,001 % |
|---|---|
| $NC-C-CO-NH_2$<br>‖<br>$N-OH$<br><br>(bekannt) | 17 |
| $NC-C-CO-NH-CO-NH-(CH_2)_5-CN$<br>‖<br>$N-C-CH_3$ | 1 |
| $NC-C-CO-NH-CO-NH-(CH_2)_5-CN$<br>‖<br>$N-O-C_2H_5$ | 9 |
| $NC-C-CO-NH-CO-NH-(CH_2)_5-CN$<br>‖<br>$N-O-C_6H_{13}-n$ | 15 |

Beispiel B

Phytophthora-Test (Tomaten) / Kurativ

Lösungsmittel 4,7 Gewichtsteile Aceton
Dispergiermittel 0,3 Gewichtsteile Alkylarylpolyglykoläther
Wasser 95 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die gennanten Zusätze enthält.

Junge Tomatenpflanzen mit 2 bis 4 Laubblättern werden mit einer wässrigen Sporensuspension von Phytophthora infestans inokuliert. Die Pflanzen bleiben 7 Studen bei 20°C und einer relativen Luftfeuchtigkeit von 100 % stehen.

Nach einer kurzen Abtrocknungszeit werden die Pflanzen mit der Spritzflüssigkeit, die in der oben angegebenen Weise hergestellt wurde, tropfnaß gespritzt und anschließend in eine Feuchtkammer mit 100 % Luftfeuchtigkeit und 18 bis 20°C Temperatur gebracht.

Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.

Wirkstoffe, Wirkstoffkonzentrationen und Ergebnisse gehen aus der folgenden Tabelle hervor:

TABELLE B
Phytophthora-Test (Tomaten) / Kurativ

| Wirkstoff | Befall in % bei einer Wirkstoffkonzentration von 0,025 % |
|---|---|
| 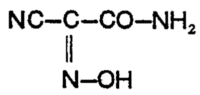 (bekannt) | 15 |
| 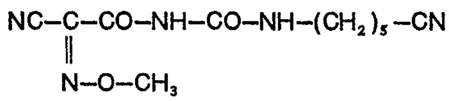 | 1 |
| 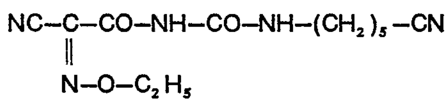 | 1 |
| 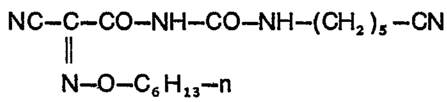 | 15 |

Beispiel C
Saatgutbeizmittel-Test / Weizensteinbrand (samenbürtige Mykose)

Zur Herstellung eines zweckmäßigen Trockenbeizmittels verstrekt man den Wirkstoff mit einem Gemisch aus gleichen Gewichtsteilen Talkum und Kieselgur zu einer feinpulverigen Mischung mit der gewünschten Wirkstoffkonzentration.

Man kontaminiert Weizensaatgut mit 5 g Chlamydosporen von Tilletia caries pro kg Saatgut. Zur Beizung schüttelt man das Saatgut mit dem Beizmittel in einer verschlossenen Glasflasche. Das Saatgut wird auf feuchtem lehm unter einer Deckschicht aus einer Lage Mull und 2 cm mäßig feuchter Komposterde 10 Tage lang im Kühlschrank bei 10°C optimalen Keimungsbedingungen für die Sporen ausgesetzt.

Anschließend bestimmt man mikroskopisch die Keimung der Sporen auf den Weizenkörnern, die jeweils it rund 100 000 Sporen besetzt sind. Der Wirkstoff ist umso wirksamer je weniger Sporen gekeimt sind.

Wirkstoffe, Wirkstoffkonzentrationen im Beizmittel, Beizmittelaufwandmengen und Keimprozente der Sporen gehen hervor aus der nachfolgenden Tabelle:

TABELLE C
Saatgutbeizmittel-Test / Weizensteinbrand

| Wirkstoffe | Wirkstoffkonzentration im Beizmittel in Gew.–% | Beizmittelaufwandmenge in g /kg Saatgut | Sporenkeimung in % |
|---|---|---|---|
| ungebeizt | – | – | >10 |
| 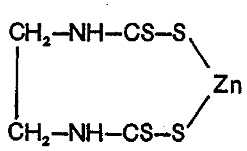 (bekannt) | 10 | 1 | 0,5 |
| 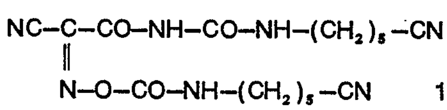 | 10 | 1 | 0,05 |

## Beispiel D

## Phytotoxizitäts-Test

| | | |
|---|---|---|
| Lösungsmittel | 4,7 | Gewichtsteile Aceton |
| Emulgator | 0,3 | Gewichtsteile Alkylarylpolyglykoläther |
| Wasser | 95 | Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält. Mit der Spritzflüssigkeit bespritzt man junge Tomaten bis zur Tropfnässe. Nach dem Abtrocknen werden die Pflanzen im Gewächshaus bei einer Temperatur von +20°C und ca. 70 % relativer Luftfeuchtigkeit aufgestellt.

Die Pflanzen werden wiederholt auf Schäden ausgewertet. Die Auswertung erfolgt nach einem Boniturschema von 1 bis 9.

1 bedeutet keine Schäden

9 bedeutet, daß die Pflanzen total geschädigt bzw. abgestorben sind.

Der Beobachtungszeitraum beträgt in der Regel 4 Tage.

Wirkstoffe, Wirkstoffkonzentration und Ergebnisse gehen aus der nachfolgenden Tabelle hervor:

### TABELLE D

Phytotoxizitäts-Test

| Wirkstoff | Schädigung bei einer Wirkstoffkonzentration von 0,3 % |
|---|---|
| 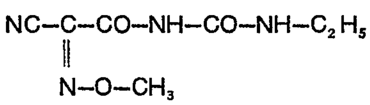 (bekannt) | 4 |
| 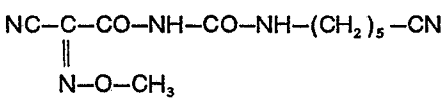 | 3 |
| 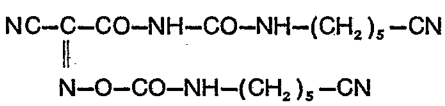 | 3 |
| 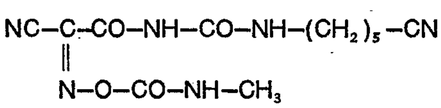 | 3 |
| 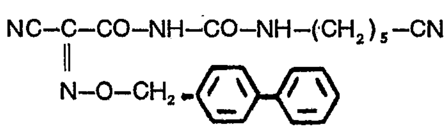 | 3 |
| 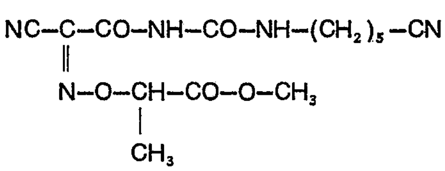 | 3 |
| 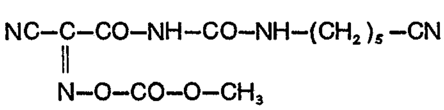 | 3 |
| 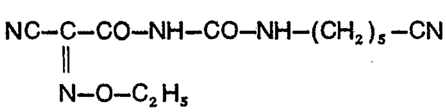 | 3 |

TABELLE D (Fortsetzung)

Phytotoxizitäts-Test

| Wirkstoff | Schädigung bei Wirkstoff-konzentration von 0,3 % |
|---|---|
| 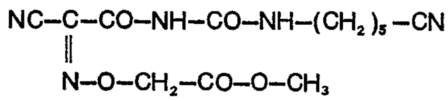 | 3 |
| 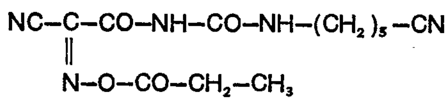 | 3 |
| 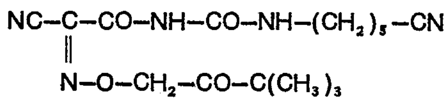 | 3 |

## Herstellungsbeispiele

### Beispiel 1

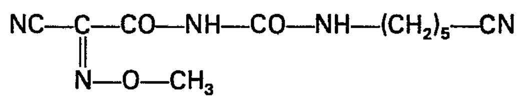

(Herstellung erfolgt gemäß Verfahren b))

500 g 1-(5-Cyanpentyl)-3-(2-cyan-2-oximino-acetyl)-harnstoff (1,99 Mol), 2 ltr. Acetonitril, 260 g Dimethylsulfat (2,06 Mol) werden bei 40°C vorgelegt. Unter gutem Rühren gibt man in etwa 22 Minuten soviel einer Lösung von 84 g Natriumhydroxid, in 440 ml Wasser gelöst, hinzu, bis ein pH-Wert von etwa 8 erreicht ist. Dann stellt man die Reaktionsmischung sofort auf einen pH-Wert von 5 ein. Durch Zugabe von Eis wird das Reaktionsprodukt ausgefällt. Es wird abgetrennt, mit Wasser salzfrei gewaschen und bei 60°C und einem Druck von 1 m Hg getrocknet. Ausbeute: 503 g 1-(5-Cyanpentyl)-3-(2-cyan-2-methoxyimino-acetyl)-harnstoff vom Fp. 92°C, das sind 95% der Theorie. Die Verbindung kann aus Wasser oder aus Isopropylalkohol umkristallisiert werden.

Vorprodukt:

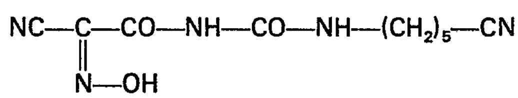

550 g (2,47 Mol) 1-(5-Cyanpentyl)-3-(2-cyanacetyl)-harnstoff, 625 g Wasser, 625 g Dioxan, 188 g techn. Natriumnitrit werden bei 50°C durch langsamen Zusatz von etwa 15 %iger Schwefelsäure auf einen pH-Wert von 5 eingestellt. Man rührt zwei Stunden, gibt dann 5 ltr. Wasser und 5 ltr. Äthylacetat hinzu. Es wird auf 2°C abgekühlt und durch weitere Zugabe von 15 %iger Schwefelsäure ein pH-Wert von 2 eingestellt. Die organische Phase wird abgetrennt und die wäßrige Phase ein zweites Mal mit 2 ltr. Äthylacetat gerührt. Die Äthylacetatlösung wird zweimal mit je 2 ltr. Wasser, welches zur besseren Trennung eine geringe Menge Natriumsulfat gelöst enthält, gewaschen und zweimal über Natriumsulfat getrocknet. Durch Zugabe von Petroläther wird das Reaktionsprodukt ausgefällt. Es wird mit einer Mischung aus Petroläther und Äthylacetat im Verhältnis 4:1 gewaschen und bei 60°C und einem Druck von 0,1 mm Hg getrocknet. Ausbeute: 504 g 1-(5-Cyanpentyl)-3-(2-cyan-2-oximino-acetyl)-harnstoff von Fp. 147°C.

Aus der Mutterlauge kann durch Einengen noch eine zweite Kristallfraktion gewonnen werden (ca. 70 g).

Zur weiteren Reinigung kann das Produkt aus Wasser, Diäthylketon oder Äthylacetat umkristallisiert werden.

Zwischenprodukt: $NC—CH_2—CO—NH—CO—NH—(CH_2)_5—CN$

90,4 g (1,06 Mol) Cyanessigsäure, 163 g (1,05 Mol) 1-(5-Cyanpentyl)-harnstoff, 200 ml trockenes Toluol werden in 35 Minuten mit 135,6 g (1,32 Mol) Essigsäureanhydrid versetzt. Man

erwärmt die Reaktionsmischung 3 Stunden auf 60 bis 67°C. Die Reaktionslösung wird bei ca. 70°C filtriert. Das Reaktionsprodukt scheidet sich beim Abkühlen aus. Es wird abgesaugt, mit Toluol gewaschen und bei 60°C und einem Druck von 0,1 mm Hg getrocknet. Ausbeute: 137,8 g 1-(5-Cyanpentyl)-3-(2-cyanacetyl)-harnstoff. Fp. 112°C. Die Verbindung kann aus Toluol, Äthylacetat oder aus Wasser umkristallisiert werden. Fp. 113,5°C.

Ausgangsprodukt: $H_2N—CO—NH—(CH_2)_5—CN$

1019 g $\omega$-Cyanpentylisocyanat werden in 4,8 ltr. trockenem Toluol gelöst. Unter Rühren wird Ammoniak über die Lösung geleitet bis nichts mehr aufgenommen wird. Das Reaktionsprodukt scheidet sich ab. Es wird abgetrennt, mit Toluol, Isopropanol und mit Wasser gewaschen und bei 60°C und einem Druck von 0,1 mm Hg getrocknet. Ausbeute: 1092 g 1-(5-Cyanpentyl)-harnstoff. Fp. 143—144°C.

(Die Verbindung läßt sich auch aus 5-Aminocapronsäurenitril und Kaliumcyanat herstellen).

Beispiel 2

NC—C—CO—NH—CO—NH—$(CH_2)_5$—CN
‖
N—O—$C_6H_{13}$-n

(Herstellung erfolgt gemäß Verfahren b))

25,1 g (0,1 Mol) 1-(5-Cyanpentyl)-3-(2-cyan-2-oximino-acetyl)-harnstoff, 100 ml Acetonitril und 17 g 1-Bromhexan werden bei 40°C vorgelegt und 13 g Äthyldiisopropylamin in 30 Min. zugetropft. Man hält noch 30 Minuten auf 40°C, gibt 1 ml Essigsäure, 400 ml Methylenchlorid und Wasser hinzu. Die Methylenchloridlösung wird dreimal mit Wasser ausgewaschen und im Vakuum eingedampft. Der Rückstand kristallisiert nach Behandeln mit Petroläther. Ausbeute 25 g 1-(5-Cyanpentyl)-3-(2-cyan-2-hexyloximino-acetyl)-harnstoff. Fp. 47°C.

Beispiel 3

NC—C—CO—NH—CO—NH—$(CH_2)_5$—CN
‖
N—O—CO—$CH_2$—$CH_3$

(Herstellung erfolgt gemäß Verfahren c))

25,1 g (0,1 Mol) 1-(5-Cyanpentyl)-3-(2-cyan-2-oximinoacetyl)-harnstoff, 100 ml Acetonitril und 9,3 g (0,1 Mol) Propionsäurechlorid werden vorgelegt und 7,9 g Pyridin zugetropft. Nach 18 Stunden wird die Reaktionsmischung filtriert und die erfindungsgemäße Verbindung durch Zugabe von Eis Ausgefällt, abgetrennt, mit Wasser gewaschen und bei 60°C und unter einem Druck von 0,1 mm Hg getrocknet. Ausbeute: 26,6 g 1-(5-Cyanpentyl)-3-(2-cyan-2-propionyl-oximino-acetyl)-harnstoff. Fp. 116,5°C.

Beispiel 4

NC—C—CO—NH—CO—NH—$(CH_2)_5$—CN
‖
N—O—CO—NH—$CH_3$

(Herstellung erfolgt gemäß Verfahren d))

25,1 g (0,2 Mol) 1-(5-Cyanpentyl)-3-(2-cyan-2-oximino-acetyl)-harnstoff, 100 ml trockenes Acetonitril und 6 g Methylisocyanat werden vorgelegt. 100 mg Triäthylendiamin werden hinzugefügt. In exothermer Reaktion entsteht 1-(5-Cyanpentyl)-3-(2-cyan-2-methylaminocarbonyl-oximino-acetyl)-harnstoff. Er wird abgesaugt, mit Acetonitril gewaschen und bei 60°C und einem Druck von 0,1 mm Hg getrocknet. Fp. 141,5—146,5°C (Zers.) IR ($CHCl_3$):CO 1805 $cm^{-1}$.

Wie in den obigen Beispielen beschrieben, lassen sich die folgenden Verbindungen der allgemeinen Formel

NC—C—CO—NH—CO—NH—$(CH_2)_5$—CN
‖ (I)
N—O—R

herstellen:

| Beispiel-Nr. | Herstellung nach Verf.-Variante | R | Fp (°C) |
|---|---|---|---|
| 5 | b | $C_2H_5$ | 105,5 |
| 6 | b | $CH(CH_3)-CO-O-CH_3$ | 126,5 |
| 7 | b | $CH_2-CO-O-CH_3$ | 115,5 |
| 8 | b | $CH_2-CO-C(CH_3)_3$ | 134 |
| 9 | b | $CH_2-CO-NH_2$ | 160 |
| 10 | b | $CH_2-CO-NH-$ 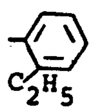 | 157,5 |
| 11 | b | $CH_2-$ 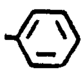 | 96 |
| 12 | b | $CH_2-$ 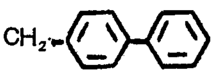 | 138 |
| 13 | b | $CH_2-$ 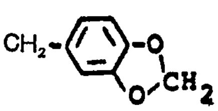 | 105 |
| 14 | c | $CO-C(CH_3)_3$ | 115 |
| 15 | c | $CO-CH_2Cl$ | 113 |
| 16 | c | $CO-O-CH_3$ | 130 |
| 17 | c | $CO-O-CH(CH_3)_2$ | 139 |
| 18 | d | $CO-NH-(CH_2)_5-CN$ | 97 IR (KBr) : ∞ 1795 $cm^{-1}$ |
| 19 | d | $CO-NH-$ 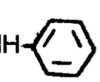 | 138,5 – 146 (Zers.) IR (KBr) : ∞ 1800 – 1805 $cm^{-1}$ |
| 20 | b | $CH_2-CH=CH_2$ | 88 |
| 21 | b | $CH_2-C\equiv CH$ | 126 |

Beispiel 22

$$NC-\underset{\underset{N-O-CH_3}{\|}}{C}-CO-NH-CO-NH-(CH_2)_5-CO-NH_2$$

Die Herstellung der Verbindung erfolgt gemäß Herstellungsverfahren b) durch Methylierung der entsprechenden Hydroxy-Verbindung, die nachstehend beschrieben wird. Fp. 155°C.

Vorprodukt:

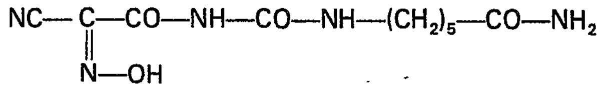

72 g 1-(5-Aminocarbonyl-pentyl)-3-(2-cyanacetyl)-harnstoff, 125 ml Wasser, 125 ml Dioxan, 23,2 g Natriumnitrit und 1 ml Essigsäure werden bei 50°C durch langsames Zutropfen von 15 %iger Schwefelsäure auf pH 4,5 bis 5 eingestellt. Man rührt während 2 Stunden bei 50°C, verdünnt mit 600 ml Wasser und 600 ml Äthylazetat, kühlt dann auf 20°C ab und stellt die Reaktionsmischung auf einen pH-Wert von 2 ein. Die Kristalle werden abgetrennt, mit Wasser gewaschen und in Vakuum getrocknet. Man erhält 61,3 g 1-(5-Aminocarbonyl-pentyl)-3-(2-cyan-2-oximino-acetyl)-harnstoff von Fp. 187—189°C.

Zwischenprodukt: $NC—CH_2—CO—NH—CO—NH—(CH_2)_5—CO—NH_2$

Dieses Zwischenprodukt wird analog dem nach Beispiel 1 angegebenen Zwischenprodukt ausgehend von 1-(5-Aminocarbonylpentyl)-harnstoff hergestellt. Fp. 164°C.

Ausgangsprodukt: $H_2N—CO—NH—(CH_2)_5—CO—NH_2$

Unter außerer Kühlung mit Eis werden 502 g (2 Mol) Phenoxycarbonylamino-capronsäure, 1,2 l Chlorbenzol, 0,6 l Äthylenchlorid, 0,4 g Antimontrichlorid und 1 g Dimethylformamid vorgelegt. Bei 9°C werden in 90 Minuten 256 g (2,15 Mol) Thionylchlorid eingetropft. Die Reaktionsmischung wird weitere 4 Stunden gerührt. Dann wird ein Teil des Lösungsmittels bis zu einer Sumpftemperatur von 31°C bei 11 Torr abdestilliert. Anschließend wird bei —20°C Ammoniak auf die Reaktionsmischung geleitet, bis nichts mehr aufgenommen wird. Nach Zugabe von 1,6 l 31 %iger wässriger Ammoniak-Lösung wird langsam zum Sieden erhitzt und ca. 8 Stunden auf Siedetemperatur gehalten. Die Kristalle werden abgetrennt, mit Wasser gewaschen und bei 70°C im Vakuum getrocknet. Ausbeute 171 g 1-(5-Aminocarbonylpentyl)-harnstoff von Fp. 208°C.

Beispiel 23

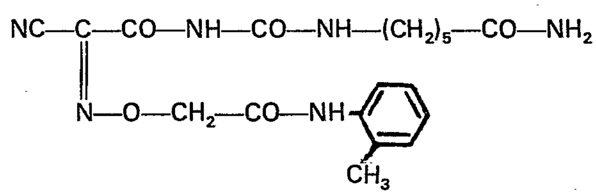

Die Herstellung der Verbindung erfolgt nach Verfahren b). Fp. 172°C.

Beispiel 24

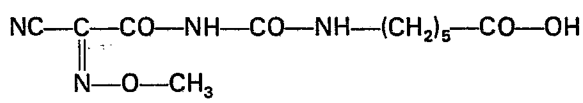

Die Herstellung erfolgt nach Verfahren b). Fp. 167°C.

Vorprodukt:

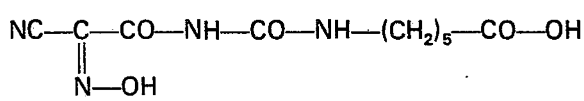

48,2 g (0,2 Mol) 1-(5-Carboxypentyl)-3-(2-cyanacetyl)-harnstoff, 50 ml Wasser, 50 ml Dioxan und 15,2 g Natriumnitrit werden auf 50°C erwärmt. Es wird sodann soviel einer Mischung aus 13 g Schwefelsäure und 90 g Wasser zugetropft, daß ein pH-Wert von 4,6 resultiert. Man hält noch 2 Stunden bei 50°C, kühlt dann auf ca. 1°C ab, gibt 400 ml Wasser hinzu und vermindert den pH-Wert durch weiteres Zutropfen von verdünnter Schwefelsäure auf 2,5. Die Kristalle werden abgetrennt, mit Wasser gewaschen und bei 60°C im Vakuum getrocknet. Die Ausbeute beträgt 51,7 g an 1-(5-Carboxypentyl)-3-(2-cyan-2-oximino-acetyl)-harnstoff von Fp. 201°C.

Zwischenprodukt: $NC—CH_2—CO—NH—CO—NH—(CH_2)_5—CO—OH$

Das Zwischenprodukt wird entsprechend den Angaben für das Zwischenprodukt nach Beispiel 1 ausgehend von 1-(5-Carboxypentyl)-harnstoff hergestellt. Fp. 158°C.

Ausgangsprodukt: $H_2N—CO—NH—(CH_2)_5—CO—OH$

260,5 g (1,04 Mol) Phenoxycarbonylamino-capronsäure werden in 500 ml Dioxan suspendiert. Unter äußerer Kühlung werden 800 ml 33 %ige wäßrige Ammoniak-Lösung zugetropft. Die Mischung wird innerhalb von 3 Stunden auf 90°C gebracht und während 6 Stunden auf dieser Temperatur gehalten. Man destilliert einen Teil des Lösungsmittels ab und säuert mit Schwefelsäure auf einen pH-Wert von 3 an. Nach Abkühlen der Mischung auf 3°C werden die Kristalle abgesaugt und mit Wasser gewaschen. Die Ausbeute beträgt 116 g 1-(5-Carboxypentyl)-harnstoff von Fp. 167—181°C.

Beispiel 25

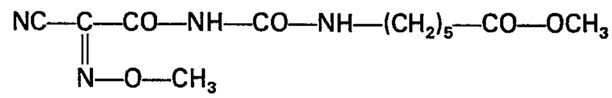

9,8 g der Verbindung gemäß Beispiel 24, 100 ml Acetonitril, 1,8 g Natriumcarbonat und 4,8 g Dimethylsulfat werden während 5 Stunden bei 70°C gehalten. Hiernach kühlt man auf 2°C ab, fügt 100 g Wasser und Eis hinzu und trennt die abgeschiedenen Kristalle ab. Man wäscht dieselben mit Wasser und erhält 9 g 1-(5-Methoxycarbonyl-pentyl)-3-(2-cyan-2-methoxyimino-acetyl)-harnstoff von Fp. 91°C.

**Patentansprüche**

1. $\omega$-Substituierte Pentyl-harnstoff-Derivate der allgemeinen Formel

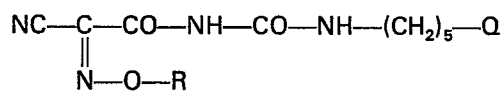

(I)

in welcher

R für $R^1$, CO—$R^2$, CO—NH—$R^3$ oder für CO—$OR^4$ steht, wobei

$R^1$ für unsubstituiertes Alkyl mit 1 bis 10 Kohlenstoffatomen steht; ferner für substituiertes Alkyl mit 1 bis 4 Kohlenstoffatomen steht, welches als Substituenten eine Vinylgruppe, eine Alkinylgruppe mit bis zu 4 Kohlenstoffatomen, eine Alkylcarbonylgruppe mit 2 bis 5 Kohlenstoffatomen, eine Alkoxycarbonylgruppe mit 2 bis 5 Kohlenstoffatomen, eine Alkenoxycarbonyl- oder Alkinoxycarbonyl-Gruppe mit 4 bis 5 Kohlenstoffatomen, eine Aminocarbonyl-, N-Alkylaminocarbonyloder N-Cycloalkylaminocarbonyl-Gruppe mit jeweils bis zu 7 Kohlenstoffatomen, oder eine N-Phenylaminocarbonylgruppe, die am Phenylrest gegebenenfalls niedere Alkylgruppen und/oder Chloratome als weitere Substituenten besitzen kann, enthält; ferner für Benzyl steht, welches im aromatischen Teil durch Methyl-, Methoxy-, Methylendioxy-, Nitro-, Trifluoromethyl-, Benzoyl-, Mono- oder Dichlorbenzoyl-, Phenyl- oder Phenoxy-Gruppen oder durch 1 bis 4 Chloratome substituiert sein kann; und

$R^2$ für einen geraden oder verzweigten, gesättigten oder ungesättigten Kohlenwasserstoffrest mit bis zu 8 Kohlenstoffatomen steht, welcher durch Chlor- oder Bromatome bzw. durch eine Cyangruppe substituiert sein kann, und

$R^3$ für einen geraden oder verzweigten Alkylrest mit bis zu 11 Kohlenstoffatomen steht, welcher durch eine Cyangruppe oder durch einen Carbonyloxyalkylrest mit bis zu 5 Kohlenstoffatomen substituiert sein kann, oder für einen Phenylrest steht, der durch Methyl-, Nitro- oder Trifluoromethyl-Gruppen bzw. durch Chloratome substituiert sein kann, und

$R^4$ für eine gesättigte oder ungesättigte aliphatische Gruppe mit bis zu 4 Kohlenstoffatomen steht, und

Q für CN, CO—$NH_2$, COOH oder CO—$OR^5$ steht, wobei

$R^5$ eine Alkyl-Gruppe mit 1 bis 4 Kohlenstoffatomen bedeutet,

2. Verfahren zur Herstellung von $\omega$-substituierten Pentyl-harnstoff-Derivaten, dadurch gekennzeichnet, daß man

a) ein 2-Cyan-2-alkoxyimino-acetamid der Formel

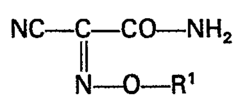

(II)

in welcher

$R^1$ die in Anspruch 1 angegebene Bedeutung besitzt,

in Gegenwart einer starken Base mit einem $\omega$-substituierten Pentylisocyanat der Formel

$$O{=}C{=}N{-}(CH_2)_5{-}Q \qquad \text{(III)}$$

in welcher

Q die in Anspruch 1 angegebene Bedeutung besitzt, umsetzt oder

b) den 1-(5-substituierten Pentyl)-3-(2-cyan-2-oximino-acetyl-harnstoff der Formel

$$NC{-}\underset{\underset{N-OH}{\|}}{C}{-}CO{-}NH{-}CO{-}NH{-}(CH_2)_5{-}Q \qquad \text{(IV)}$$

in welcher

Q die in Anspruch 1 angegebene Bedeutung besitzt,

in Gegenwart eines Säurebindemittels, bzw. ein Alkali-, Erdalkali- oder Ammoniumsalz des Harnstoffs der Formel IV mit einem Alkylierungsmittel der Formel

$$X{-}R^1 \qquad \text{(V)}$$

in welcher

$R^1$ die in Anspruch 1 angegebene Bedeutung besitzt, und

X für eine "Abgangsgruppe" wie Chlor, Brom, Jod, Akoxy-, Alkyl- bzw. Arylsulfonyl steht, und ferner für die Gruppe $(CH_3O{-})_2PO{-}O$ steht, wenn $R^1$ für Methyl steht,

umsetzt, bzw.

c) den Harnstoff der Formel IV mit einem Säurederivat der Formel

$$T{-}CO{-}R^2 \qquad \text{(VI)}$$

in welcher

$R^2$ die in Anspruch 1 angegebene Bedeutung besitzt und

T für Chlor, Brom, Jod oder die Gruppe $R^2{-}CO{-}O$ steht, umsetzt, bzw.

d) den Harnstoff der Formel IV mit einem Isocyanat der Formel

$$OCN{-}R^3 \qquad \text{(VII)}$$

oder mit einem Carbamidsäurehalogenid der Formel

$$Y{-}CO{-}NH{-}R^3 \qquad \text{(VIII)}$$

in welchen Formeln

$R^3$ die in Anspruch 1 genannte Bedeutung besitzt und

Y für Chlor, Brom oder Jod steht,

umsetzt, bzw.

e) den Harnstoff der Formel IV mit einem Kohlensäureester der Formel

$$Z{-}CO{-}O{-}R^4 \qquad \text{(IX)}$$

in welcher

$R^4$ die in Anspruch 1 angegebene Bedeutung besitzt und

Z für Chlor oder den Rest $R^4{-}O{-}CO{-}O$ steht, umsetzt;

und weiterhin gegebenenfalls diejenigen Verbindungen der Formel I, in denen R für $R^1$ und Q für die Gruppe $CO{-}OR^5$ steht, dadurch erhält, daß man gemäß einer weiteren Verfahrensvariante

f) eine nach Verfahren b) synthetisierte Carbonsäure der Formel

$$NC{-}\underset{\underset{N-O-R^1}{\|}}{C}{-}CO{-}NH{-}CO{-}NH{-}(CH_2)_5{-}CO{-}OH \qquad \text{(X)}$$

in welcher

$R^1$ die in Anspruch 1 angegebene Bedeutung besitzt,

in Gegenwart eines Säurebindemittels, bzw. eines Alkali-, Erdalkali- oder Ammonium-Ions, mit einem Alkylierungsmittel der Formel

$$X{-}R^5 \qquad \text{(XI)}$$

in welcher

X die oben und $R^5$ die in Anspruch 1 genannte Bedeutung besitzen,

umsetzt.

3. Fungizide Mittel, gekennzeichnet durch einen Gehalt an mindestens einem $\omega$-substituierten Pentyl-harnstoff-Derivat gemäß Anspruch 1.

4. Verwendung von $\omega$-substituierten Pentyl-harnstoff-Derivaten gemäß Anspruch 1 zur Bekämpfung von Pilzen.

5. Verfahren zur Herstellung von fungiziden Mitteln, dadurch gekennzeichnet, daß man $\omega$-substituierte Pentyl-harnstoff-Derivate gemäß Anspruch 1 mit Streckmitteln und/oder oberflächenaktiven Mitteln vermischt.

## Claims

1. $\omega$-substituted pentyl-urea derivatives of the general formula

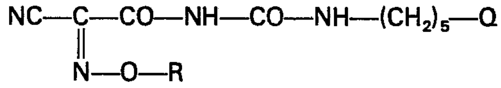

(I)

in which

R represents $R^1$, CO—$R^2$, CO—NH—$R^3$ or CO—$OR^4$, wherein

$R^1$ represents unsubstituted alkyl with 1 to 10 carbon atoms; or represents substituted alkyl with 1 to 4 carbon atoms, which contains, as a substituent, a vinyl group, an alkynyl group with up to 4 carbon atoms, an alkylcarbonyl group with 2 to 5 carbon atoms, an alkoxycarbonyl group with 2 to 5 carbon atoms, an alkenoxycarbonyl or alkynoxycarbonyl group with 4 to 5 carbon atoms, an aminocarbonyl group, an N-alkylaminocarbonyl or N-cycloalkylaminocarbonyl group with up to 7 carbon atoms in either case or a N-phenylaminocarbonyl group, which can optionally have lower alkyl groups and/or chlorine atoms as further substituents on the phenyl radical; or represents benzyl, which can be substituted in the aromatic part by methyl, methoxy, methylenedioxy, nitro, trifluoromethyl, benzoyl, monochlorobenzoyl, dichlorobenzoyl, phenyl or phenoxy groups or by 1 to 4 chlorine atoms; and

$R^2$ represents a straight-chain or branched, saturated or unsaturated hydrocarbon radical with up to 8 carbon atoms, which can be substituted by chlorine or bromine atoms or by a cyano group,

$R^3$ represents a straight-chain or branched alkyl radical with up to 11 carbon atoms, which can be substituted by a cyano group or by an alkoxycarbonyl radical with up to 5 carbon atoms, or represents a phenyl radical, which can be substituted by methyl, nitro or trifluoromethyl groups or by chlorine atoms, and

$R^4$ represents a saturated or unsaturated aliphatic group with up to 4 carbon atoms and

Q represents CN, CO—$NH_2$, COOH or CO—$OR^5$,

wherein

$R^5$ denotes an alkyl group with 1 to 4 carbon atoms.

2. A process for the preparation of $\omega$-substituted pentyl-urea derivatives characterised in that

(a) a 2-cyano-2-alkoxyimino-acetamide of the formula

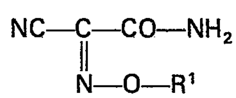

(II),

in which

$R^1$ has the meaning stated in claim 1,

is reacted with an $\omega$-substituted pentyl isocyanate of the formula

$$O{=}C{=}N{-}(CH_2)_5{-}Q \quad (III),$$

in which

Q has the meaning stated in claim 1,

in the presence of a strong base or

(b) the 1-(5-substituted pentyl)-3-(2-cyano-2-hydroximino-acetyl-urea of the formula

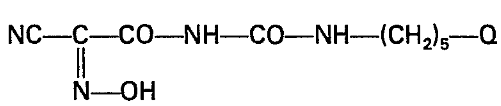

(IV),

in which

Q has the meaning stated in claim 1,

is reacted in the presence of an acid-binding agent, or an alkali metal salt, alkaline earth metal salt or

ammonium salt of the urea of the formula (IV), with an alkylating agent of the formula

$$X—R^1 \quad (V),$$

in which

$R^1$ has the meaning stated in claim 1 and

X represents a "leaving group" such as chlorine, bromine, iodine, alkoxy-, alkyl- or arylsulphonyl and also represents the group $(CH_3O—)_2PO—O$ if $R^1$ represents methyl, or (c) the urea of the formula (IV) is reacted with an acid derivative of the formula

$$T—CO—R^2 \quad (VI),$$

in which

$R^2$ has the meaning stated in claim 1 and

T represents chlorine, bromine, iodine or the group

$$R^2—CO—O,$$

or (d) the urea of the formula (IV) is reacted with an isocyanate of the formula

$$OCN—R^3 \quad (VII)$$

or with a carbamoyl halide of the formula

$$Y—CO—NH—R^3 \quad (VIII),$$

in which formulae

$R^3$ has the meaning stated in claim 1 and

Y represents chlorine, bromine or iodine, or

(e) the urea of the formula (IV) is reacted with a carbonic acid ester of the formula

$$Z—CO—O—R^4 \quad (IX),$$

in which

$R^4$ has the meaning stated in claim 1 and

Z represents chlorine or the radical $R^4—O—CO—O$;

and furthermore those compounds of formula I in which R represents $R^1$ and Q represents the group $CO—OR^5$ are optionally obtained by reacting according to a further process variant

(f) a carboxylic acid of the formula

$$\begin{array}{l} NC—\underset{\underset{\displaystyle N—O—R^1}{\|}}{C}—CO—NH—CO—NH—(CH_2)_5—CO—OH \end{array} \quad (X),$$

in which

$R^1$ has the meaning stated in claim 1,

which carboxylic acid has been synthesized according to process b),

with an alkylating agent of the formula

$$X—R^5 \quad (XI),$$

in which

X has the above-mentioned meaning and

$R^5$ has the meaning stated in claim 1,

in the presence of an acid-binding agent or of an alkali metal ion, alkaline earth metal ion or ammonium ion.

3. Fungicidal compositions characterised by a content of at least one $\omega$-substituted pentyl-urea derivative according to claim 1.

4. The use of $\omega$-substituted pentyl-urea derivatives according to claim 1 for combating fungi.

5. A process for the preparation of fungicidal compositions, characterised in that $\omega$-substituted pentyl-urea derivatives according to claim 1 are mixed with extenders and/or surface-active agents.

**Revendications**

1. Dérivés de pentyl-urée ω-substitués de formule générale:

$$\begin{array}{l} NC-C-CO-NH-CO-NH-(CH_2)_5-Q \\ \qquad \| \\ \qquad N-O-R \end{array} \qquad (I)$$

dans laquelle

R représente $R^1$, $CO-R^2$, $CO-NH-R^3$ ou $CO-OR^4$,

$R^1$ représente un alcoyle non substitué ayant 1 à 10 atomes de carbone, en outre un alcoyle substitué ayant 1 à 4 atomes de carbone qui contient comme substituants un groupe vinyle, un groupe alcinyle ayant jusqu'à 4 atomes de carbone, un groupe alcoylcarbonyle ayant 2 à 5 atomes de carbone, un groupe alcoxycarbonyle ayant 2 à 5 atomes de carbone, un groupe alcénoxycarbonyle ou alcinoxycarbonyle ayant 4 à 5 atomes de carbone, un groupe aminocarbonyle, N-alcoylaminocarbonyle ou N-cycloalcoylaminocarbonyle ayant chaque fois jusqu'à 7 atomes de carbone, ou un groupe N-phénylaminocarbonyle qui peut contenir sur le radical phényle éventuellement des groupes alcoyle inférieurs et/ou des atomes de chlore comme autres substituants, en outre benzyle qui peut être substitué dans la partie aromatique par des groupes méthyle, méthoxy, méthylènedioxy, nitro, trifluorométhyle, benzoyle, mono- ou dichlorobenzoyle, phényle ou phénoxy ou par 1 à 4 atomes de chlore et

$R^2$ représente un radical hydrocarboné à chaîne droite ou ramifiée, saturé ou non saturé, ayant jusqu'à 8 atomes de carbone, qui peut être substitué par des atomes de chlore ou de brome ou par un groupe cyano, et

$R^3$ représente un radical alcoyle à chaîne droite ou ramifiée ayant jusqu'à 11 atomes de carbone, qui peut être substitué par un groupe cyano ou par un radical carbonyloxyalcoyle ayant jusqu'à 5 atomes de carbone, ou un radical phényle qui peut être substitué par des groupes méthyle, nitro ou trifluorométhyle ou par des atomes de chlore, et

$R^4$ représente un groupe aliphatique saturé ou non saturé ayant jusqu'à 4 atomes de carbone et

Q représente CN, $CO-NH_2$, COOH ou $CO-OR^5$

$R^5$ signifiant un groupe alcoyle ayant 1 à 4 atomes de carbone.

2. Procédé de préparation de dérivés de pentyl-urée ω-substitués, caractérisé en ce que

a) on fait réagir une 2-cyano-2-alcoxyimino-acétamide de formule

$$\begin{array}{l} NC-C-CO-NH_2 \\ \qquad \| \\ \qquad N-O-R^1 \end{array} \qquad (II)$$

dans laquelle

$R^1$ possède la signification indiquée à la revendication 1,

en présence d'une base forte avec un pentyl-isocyanate ω-substitué de formule

$$O=C=N-(CH_2)_5-Q \qquad (III)$$

dans laquelle

Q possède la signification indiquée dans la revendication 1, ou

b) on fait réagir la 1-(pentyl 5-substitué)-3-(2-cyano-2-oximino-acétyl)-urée de formule:

$$\begin{array}{l} NC-C-CO-NH-CO-NH-(CH_2)_5-Q \\ \qquad \| \\ \qquad N-OH \end{array} \qquad (IV)$$

dans laquelle

Q possède la signification indiquée à la revendication 1,

en présence d'un agent fixateur d'acide ou un sel alcalin, alcalino-terreux ou ammonique de l'urée de formule IV avec un agent d'alcoylation de formule:

$$X-R^1 \qquad (V)$$

dans laquelle

$R^1$ possède la signification indiquée à la revendication 1 et

X représente un "groupe clivable" tel que chlore, brome, iode, alcoxy, alcoyl- ou arylsulfonyle et en outre représente le groupe $(CH_3O-)_2PO-O$ lorsque $R^1$ représente un méthyle, ou

c) on fait réagir l'urée de formule (IV) avec un dérivé acide de formule:

$$T-CO-R^2 \qquad (VI)$$

dans laquelle
$R^2$ possède la signification indiquée à la revendication 1 et
T représente du chlore, du brome, de l'iode ou le groupe $R^2$—CO—O, ou
d) on fait réagir l'urée de formule IV avec un isocyanate de formule:

$$OCN—R^3 \qquad (VII)$$

ou avec un halogénure de carbamyle de formule:

$$Y—CO—NH—R^3 \qquad (VIII)$$

formules dans lesquelles
$R^3$ possède la signification indiquée à la revendication 1 et
Y représente du chlore, du brome ou de l'iode, ou
e) on fait réagir l'urée de formule IV avec un ester d'acide carbonique de formule:

$$Z—CO—O—R^4 \qquad (IX)$$

dans laquelle
$R^4$ possède la signification indiquée à la revendication 1 et
Z représente du chlore ou le radical $R^4$—O—CO—O et en ce qu'en outre on obtient éventuellement les composés de formule I où R représente $R^1$ et Q le groupe CO—$OR^5$ quand on fait réagir selon une autre variante opératoire
f) un acide carboxylique synthétisé salon le procédé b), de formule:

$$\begin{array}{l} NC—\underset{\underset{\displaystyle N—O—R^1}{\|}}{C}—CO—NH—CO—NH—(CH_2)_5—CO—OH \end{array} \qquad (X)$$

dans laquelle
$R^1$ possède la signification indiquée à la revendication 1,
en presénce d'un agent fixateur d'acide, ou d'un ion alcalin, alcalino-terreux ou ammonium, avec un agent d'alcoylation de formule:

$$X—R^5 \qquad (XI)$$

dans laquelle
X possède la signification indiquée plus haut et $R^5$ celle indiquée à la revendication 1.

3. Agents fongicides, caractérisés par une teneur en au moins un dérivé de pentyl-urée $\omega$-substitué selon la revendication 1.

4. Utilisation de dérivés de pentyl-urée $\omega$-substitués selon la revendication 1 pour combattre les champignons.

5. Procédé de fabrication d'agents fongicides, caractérisé en ce qu'on mélange des dérivés de pentyl-urée $\omega$-substitués selon la revendication 1 avec des agents de dilution et/ou des agents tensionactifs.